# EUROPEAN PATENT SPECIFICATION

(11) **EP 2 812 060 B1**
(45) Date of publication and mention of the grant of the patent: **03.02.2016**
(21) Application number: 13712361.8
(22) Date of filing: 06.02.2013
(51) Int. Cl.: A61M 16/04

(54) **Tracheal aid**
Trachealhilfe
Prothèse trachéale

(30) Priority: 07.02.2012 IT VI20120030
(43) Date of publication of application: 17.12.2014
(73) Proprietor: Checcacci Carboni, Stefano, 36010 Monticello Conte Otto (IT); Turato, Fabio, 36050 Quinto Vicentino (IT)
(72) Inventor: Checcacci Carboni, Stefano, 36010 Monticello Conte Otto (IT); Turato, Fabio, 36050 Quinto Vicentino (IT)
(74) Representative: Trentin, Michele
(86) International application number: PCT/IB2013/050980
(87) International publication number: WO 2013/118059

(56) References cited:
- WO-A1-02/47748
- DE-A1- 2 120 164
- DE-B- 1 166 977
- DE-C1- 10 118 605
- US-A- 4 840 172
- US-A- 5 392 774
- US-A1- 2005 229 933

## Description

### Field of the invention

The present application is generally applicable to the medical field and concerns medical devices for the management of the patient airways.

In particular, the present invention relates to a tracheal aid usable for the assisted ventilation of patients under anesthesia or, for example, with respiratory crises.

### Background art

It is known that there are several situations in which people can have respiratory distress. Typically this takes place as a result of a trauma, but it is a phenomenon which can be induced in a patient that must be operated and is then anesthetized.

In all such cases, the patient is rescued by applying devices for assisted ventilation which may be constituted by a ball (said Ambu) or by a medical ventilator machine.

These devices must be able to access the lungs and this is done through tubes that affect the airways.

The best-known type of tubes is the endotracheal tubes. An endotracheal tube is typically made by plastic material and it is inserted into the trachea of the patient usually through the use of a laryngoscope and through the mouth, even though there are tubes for naso-tracheal intubation.

The tubes have generally circular section and may have either a predefined fixed shape or they can be flexible: the latter, to be inserted, must be modeled through a stiff wire, said spindle, which will be removed upon completion of the insertion.

The proximal portion of the tube, that protrudes from the mouth, has a link to be connected to the artificial ventilation system.

The same tube is provided with a small balloon disposed external to the tube and near the distal end. This balloon, also said cuff, can be inflated from the outside, through an injection of air into a suitable duct, so as to perfectly adhere to the tracheal wall: in this case it is said that the tube is cuffed. This prevents the entry of liquids, for example gastric liquids, in the lungs so securing the airway. It also avoids that the air can come out and also stabilizes the tube in the right location.

The endotracheal intubation process, though being the most safe process for the patient, it is however difficult to apply. In fact, it requires the inspection of the airways because the "blind" insertion means little success chance or zero success chance. As is known, in fact, next to the trachea there is the esophagus access and this is the most direct access from both the mouth or the nose. An entry without inspection therefore involves high possibility to access to the esophagus.

Endotracheal intubation is therefore generally performed by the aid of the laryngoscopy, a technique according to which is used a laryngoscope to visualize the epiglottis. The tube is so inserted with a direct vision. This technique can usually be performed only in patients in a coma state, ie generally when they are unconscious, or under general anesthesia or local anesthesia. In certain cases it is administeed a short duration sedative followed by a paralyzing curare.

Therefore, such procedures are performed with the patient not be able to breathe, ie with the patient in apnea state. It is evident the difficult situation where it is necessary to speed up the procedure.

For certain patients the visibility of the airway is physically complicated. For this reason it is generally used a fiberscope which allows to have a close up vision of the airways even in difficult cases. In particular, the insertion of the fiberscope into the trachea before the tube allows not only to carry out this operation "on sight", but also to use the fiberscope as mechanical guide for the endotracheal tube. However, even in this case, as in the preceding cases, the procedure is performed with the patient in apnea state.

Moreover, in all cases the operator must be expert in those procedures because, on the contrary, despite the available tools the intubation procedure would be under risk: there would have high possibility to insert the tube into the esophagus instead into the trachea.

Also the health state of the patient complicate the procedures. In the case, for example, of a patient subject to strong trauma, his rapid immobilization can be really complicated. If the trauma regards the face of the patient, the inspection of the airway can be seriously hampered. The presence of large amounts of blood or gastric reflux can worsen the situation and make almost impossible the endotracheal intubation, especially when it has to act in the location where the trauma occurred instead in appropriate medical centre.

Moreover, ehen curare is used the use of the fiberscope is contraindicated. The paralysis of the airways muscles makes difficult to identify them.

In addition to the failure possibility, endotracheal intubation also raises the risk of making further damage to the patient. In particular, there is the possibility to damage the dental arch, especially with the use of the laryngoscope, and the vocal cords of the patient.

Since it was known that these problems, especially when it is urgent to act on a traumatized patients, make it difficult to perform the endotracheal intubation, it has been available many tools for the assisted ventilation that are known with the name of overglottis aids.

Typically it is a tube provided with a distal inflatable cuff, and it can be inserted in the pharynx. The easy application of it, therefore, is due to the fact that it must not be inserted into the trachea. Its use is also due to a lower pain and coughing incidence then the endotracheal tube which, however, irritate the trachea. The insertion is easier requiring no additional tools.

The overglottis ais is useful in situations where the victim of a trauma is, for example, immobilized and in a sitting position, with suspected cervical spine injury, ie in cases where is contraindicated the extension of the head to keep open the airways and proceed with endotracheal intubation. Obviously, the use of the overglottis aid is recommended when the endotracheal intubation has been tried without success.

This aid, however, does not protect the lungs from the risk of aspiration of fluid from the esophagus because it not occluded this way. Therefore, it is not recommended in those patients with which there is a risk of complications such as, for example, patients who ate. The only protection it provides is for liquids coming from the overglottis area, ie nose and mouth.

Since the lacks of protection from liquids coming from the esophagus, often the overglottis aid is used, because of its ease, as an urgent aid, but it is then replaced, when the patient is disposed in a position and in an environment more comfortable, by the endotracheal tube. In fact, the two aids are incompatible with each other.

Even the application of the overglottis aid, however, requires experience by the rescuer to avoid mistakes on its positioning.

Replacing it with endotracheal intubation, also have strong criticality. During this operation, in fact, the patient is in apnea state. Obvious are the risks in subjecting the patient to a further period of absence of oxygenation.

The possible presence of liquids, such as blood, in the mouth or falling from the nose can complicate the endotracheal intubation and make it difficult or impossible. The recovery of the overglottis aid may be, in such cases, impossible.

All these difficulties could require tracheotomy incision surgery which consists on the incision of the trachea to open an alternative airway to the natural one. Obviously, this is a particularly invasive solution for the patient and, therefore, it must be avoided unless it is impossible to intervene in any other way.

The International patent application WO 02/47748 A1 is also known. It refers to a bronchial ventilation device. It enables selective ventilation of the left and right bronchi. It is so clear that the object described in D1 is not a tracheal aid, but a bronchial aid so having different needs and a different structure. If used as tracheal aid, it have the same drawbacks listed above.

### Disclosure of the invention

Object of the present invention is to at least partially overcome the drawbacks highlighted above by providing a tracheal aid that is easy to apply on any patient in whatever state it is.

In particular, an object of the present invention is to provide a tracheal aid that is usable with success even by unskilled users.

Another object of the invention is to make available a tracheal aid which is usable both as a defensive overglottis aid that as an endotracheal tube avoiding the replacement procedure described above.

In particular, one object of the invention is that the tracheal aid can be used as overglottis aid or as endotracheal tube without removing it from its position and allowing the also repeated transition from one configuration to the other.

A further object is that the tracheal aid of the present invention allows to protect the airway from liquids coming from the esophagus even when used as overglottis aid.

Another object is to provide a tracheal aid that avoids apnea periods for the patient even in the case of transition from the use as an overglottis aid to use as endotracheal tube and vice versa.

Such objects, and others which will become apparent hereinafter, are achieved by a tracheal aid in accordance with one or more of the following claims which are an integral part of the present patent.

In particular, the above tracheal aid may comprises at least one first tracheal tube having a proximal end connectable to a device for the assisted respiration of a patient and then protruding from its mouth. The distal end may be inserted on an orifice. The latter typically could be the trachea or the larynx.

Externally to the first tube may be present at least one first inflatable cuff for close the orifice in which may be inserted the first tube.

According to one aspect of the invention, the tracheal aid may comprise at least one second tracheal tube having the distal end can be inserted also on an orifice, typically the trachea or the esophagus. Similarly to the first tube, the second tube may also be externally provided by at least one inflatable cuff for close the orifice in which the second tube may be inserted.

According to another aspect of the invention, the tracheal aid may also include at least one junction element stably coupled to the first tube and the second tube. This junction element may also be flexible to allow the distal end of the first tube to be inserted on an orifice different from that in which is inserted the distal end of the second tube. In other words, the junction element may allow the distal end of the first tube to be inserted on the trachea when the distal end of the second tube is inserted on the esophagus. In particular, to allow that, the junction element is stably coupled with a distal end portion of the first tube and with a proximal end portion of the second tube.

An aid so structured, as it will seen more in detail, may be successfully applied to the patient even without inspection of the airways. In particular, the tracheal aid of the invention can be advantageously used as an overglottis aid or as an endotracheal tube in both cases saving the lungs from liquids coming not only from the nose and mouth, but also from the esophagus.

In fact, during the insertion of the aforementioned aid if the second tube, which is that furthest from the end connectable to the devices for the assisted respiration, will be inserted into the esophagus, the inflation of the second cuff may close it preventing the reflux of liquid towards the trachea.

The first tube may then be arranged as an overglottis aid, ie may be disposed in the larynx with the first cuff inflated so as to close it. The endotracheal aid of the invention, therefore, may take the configuration of an overglottis aid with the advantage that the esophagus may be closed preventing the transit of fluids to the trachea.

The junction element, being flexible, will also allow to insert the first tube into the trachea deflating the first cuff and proceeding to the correct insertion also with the possible help of a fiberscope which acts as a guide. In other words, from a configuration as a overglottis aid, the aid of the invention may be transformed into an endotracheal tube. This transformation may be reversible at any time.

An evident advantage is, therefore, to have a single aid that can be used as overglottis aid or as an endotracheal tube. Another advantage is that these two aids, being made by a single tracheal aid, have been made compatible with each other allowing the transition from one to the other without having to extract the aid from the patient.

Since both the esophagus and the larynx can be closed during the insertion of the fiberscope into the trachea, the most complicated part of the intubation procedure may be advantageously carried out during the assisted respiration of the patient. In other words, the aid of the invention at least will minimize the apnea period of the patient in the transition from the configuration as overglottis aid to the configuration as endotracheal tube. Even the opposite transitino can be done by limiting or nullifying the apnea period of the patient.

If the second tube was fortuitously inserted into the trachea, the tracheal aid of the invention can be configured directly as an endotracheal tube.

Therefore, advantageously, the aid of the invention may be used "blindly", ie without the inspection of the airways and by unskilled users since in any case allow the best result, that is a fast respiratory care for the patient.

### Brief description of the drawings

Further features and advantages of the invention will become more apparent from the detailed description of a preferred, non exclusive embodiment of a tracheal aid according to the invention presented by way of illustrating and non-limiting examples in connection with the accompanying drawing in which:

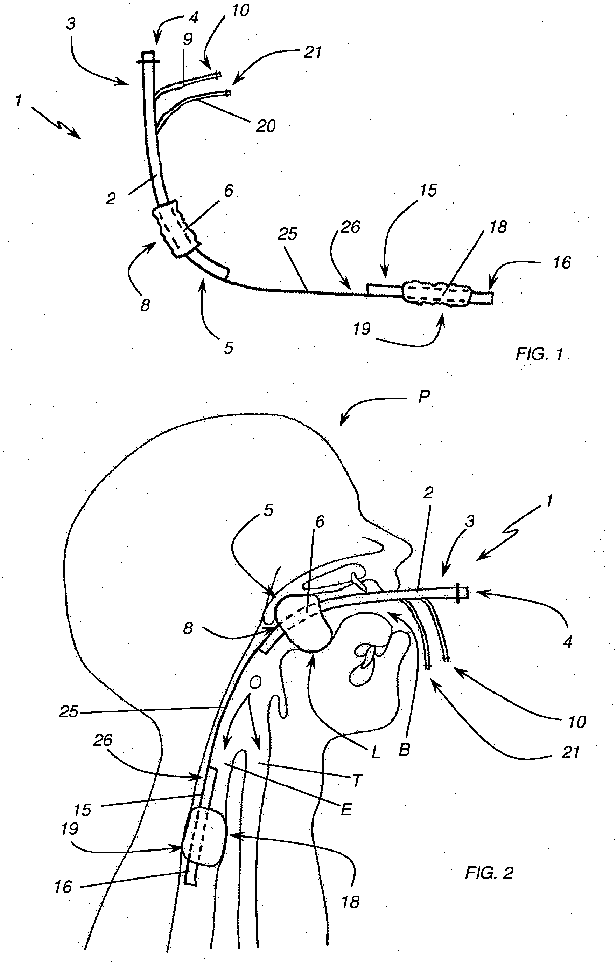

the FIG. 1 represents a tracheal aid according to the invention;
the FIGS. from 2 to 5 represent some operative instants of the tracheal aid shows in the FIG. 1.

### Detailed description of a preferred embodiment

With reference to the cited figures, and in particular to Figs. 1 and 2, it is described a tracheal aid **1** provided to medical and paramedical staff. The aid **1** being described is suitable to be inserted through the mouth **B.** However this should not be considered limiting for the present invention that could be of the type insertable by the nose.

It comprises a first tracheal tube **2** having a proximal end **3** to be arranged protruding from the mouth **B** of the patient **P.** Said end is used for connection to a device for the assisted respiration that is not an object of this patent. In this sense, the same end has a tight junction **4** to supply air and, more generally, fluids to the patient **P** for his breathing.

Typically, devices such as balloons Ambu, manually activated, or ventilation machines they are connected to the tight junction **4.**

The remaining part of the first tube **2** is inserted into the mouth **B** of the patient and at least the distal end **5** can be inserted on an orifice **O** as, for example, the larynx L or the trachea **T.**

A first inflatable cuff **8** is stably arranged around a portion **6** of the first tube **2,** the usefulness of said first cuff **8** will be further explained hereinafter. For its inflating it is connected to a first pipe **9** which ends outside the patient and which also have a junction **10.** Through this junction **10** is possible to proceed with the inflating using, for example, a syringe.

The portion **6** of the first pipe **2** around which is arranged the first cuff **8** is usually near the distal end **5** of the first tube **2,** but this should not be considered limiting for different embodiments of the invention.

According to one aspect of the invention, the tracheal aid **1** comprises a second tracheal tube **15** having the distal end **16** which can be inserted on an orifice **O** as, for example, the trachea **T** or the esophagus **E.**

As specified, both tubes **2** and **15** are of tracheal type, ie they have shape and size such that they can be inserted into the trachea **T.** Obviously, if the aid is suitable to be inserted via nose, the size will be appropriate.

In any case, typically the tubes are cylindrical and made by biocompatible materials. Generally, they are also flexible, but this should not be considered limiting for different embodiments by which they have a predefined and not changeable shape.

Even in this case, around a portion **18** of the second tube **15** there is a second inflatable cuff **19,** the usefulness of which will be further explained hereinafter. For its inflating it is connected to a second pipe **20** which terminates outside the patient and which also provided with a junction **21.** Through such a junction **21** it is possible to proceed with the inflating of the second cuff **19** separately from the first cuff **8.**

The portion **18** of the second tube **15** around which is disposed the second cuff **19** is usually near the distal end **16** of the second tube **15,** but this should not be considered limiting for different embodiments of the invention.

According to another aspect of the invention, the first tube **2** and the second tube **15** are firmly connected by means of a junction element **25.** The latter is interposed therebetween so that the first pipe **2,** the second tube **15** and the junction member **25** are substantially aligned with each other. The junction element **25** is also flexible to allow the distal ends **5** and **16** to be inserted on different orifices **O.** In other words, the flexibility of the junction element **25** allows to insert the two tracheal tubes **2** and **15** the first into the trachea **T** and the second into the esophagus **E.**

The junction element **25** is connected to the distal end **portion 5** of the first tube **2** and the proximal end portion **26** of the second tube **15.** In particular, one end of the junction element **25** is connected with the distal end portion **5** of the first tube **2** and the second end of the junction element **25** is connected with the proximal end portion **26** of the second tube **15.**

Advantageously, therefore, the tracheal aid **1** of the invention can affect simultaneously the trachea **T** and the esophagus **E.** This is a new fact that have more advantages.

Both tubes **2** and **15,** in fact, have respective inflatable cuff **8** and **19** which allow to close different channels.

From an operative point of view, the user insert the aid **1** of the invention in the mouth **B** of the patient **P.** Without inspection of the airways and without the use of the laryngoscope, as represented in Fig. 2, the second tube **15** will be inserted into the esophagus **E** with high probability. In case the user wants to use the aid **1** as an overglottis aid, he continues with the insertion until the first cuff **8** connected to the first tube **2** does not reach the larynx L. This position may be indicated by a special notch on the first pipe **2** in order to facilitate this operation by the user, but this, as we will see later, is not necessary.

Next, the user can proceed with the inflation of both cuffs **8** and **19.** By this operation it shall then closed the esophagus **E** and the larynx **L.** Advantageously, therefore, not only the aid **1** of the invention realizes a overglottis aid, but this aid is more secure than the equivalent ones of the known art because it allows the closure of the esophagus **E** preserving the lungs from stomach acid or by any other type of regurgitation of the patient **P.** In other words, the overglottis aid obtained is safer than the known ones.

The user can then connect the device for assisted respiration to the tight junction **4** presents at the proximal end **3** of the first tube **2** successfully ventilating the patient **P.**

In order to allow the deflating of gas or fluid present in the stomach, the aid **1** of the invention also includes an additional conduit, not represented in the figures, which extends from the proximal end **3** of the first tube **2** to the distal end **16** of the second tube **15** for putting in fluid communication the two said ends **3, 16.** Consequently are in fluid communication the stomach of the patient **P** and the external environment and through this duct additional gases or fluids that may be present in the stomach can be expelled improving the conditions of the patient **P.**

According to another aspect of the invention, the flexibility of the junction element **25** could make difficult the management of the insertion of the two tubes **2, 15** on the patient **P.** For this reason the aid 1 also comprises a semirigid element **30** disposed inside the first tube **2** and the second tube **15.** It is extractable from these tubes **2, 15** and its functionality is to maintain mutually aligned the tubes **2, 15** during their insertion into the patient **P.** After the insertion, the semirigid element **30** is extracted so as to leave the junction element **25** free to move.

Moreover, the fact that the element **30** is semirigid allows not only to maintain aligned the two tubes **2, 15,** but also to shape the aid 1 allowing to adapt it to the patient P simplifying the insertion of the aid **1.**

According to a further aspect of the invention, the semirigid element **30** has a system for indicating the penetration depth of the aid **1** in the patient **P.** It is typically formed by a graduated scale and it allows to replace the previously mentioned notches on the first tube **2.** Since the tracheal aid **1** may have different sizes for better adapt to different patients **P,** the semirigid element **30** thus obtained can instead be the same for all the tracheal aid 1 allowing to save the production costs and the management of the material stock.

It was said before that the tracheal aid **1** can be designed to be used via the nose rather than by mouth of patients affect by specific diseases or trauma. In this case, it underlines the further advantage of the invention to provide an overglottis aid insertable via nose, that is a currently not existing solution.

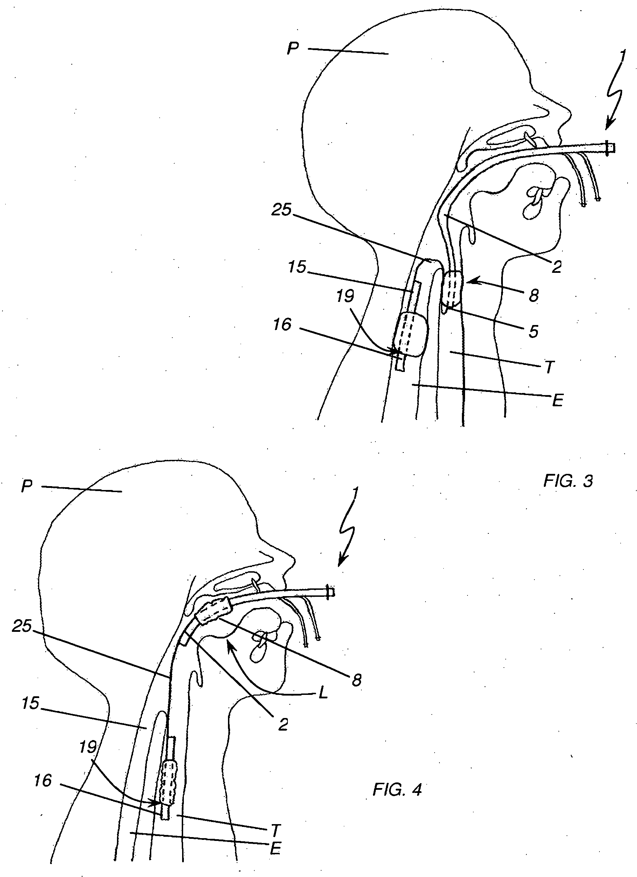

If it is necessary to proceed with the intubation of the patient **P,** unlike the prior art in the present case it is not necessary to remove the aid **1.** It is sufficient to proceed, as shown in Fig. 3, to insert the distal end **5** of the first tube **2** in the trachea **T.** This operation can be advantageously carried out without removing the second tube **15** because the articulation and flexibility of the junction element **25** that allows the distal end **5** of the first tube **2** to be inserted on an orifice **O** different from that in which is inserted the second tube **15** (typically the trachea **T** for the first tube **2** and the esophagus to the second tube **15).**

Operatively it is possible to proceed by inserting a fiberscope in the first tube **2,** by identifying the trachea **T,** by inserting the fiberscope into the trachea **T** in order to use it as a guide, by deflating the first cuff **8** and inserting the first tube **2** into the trachea **T** using the fiberscope. Obviously the use of the fiberscope is optional and this procedure must not be considered limitative for the present invention because the same operation can be executed by a more traditional laryngoscope.

After insertion into the trachea **T,** there is the inflation of the first cuff **8** finishing the transformation of the aid **1** from an overglottis aid to an endotracheal tube.

It is therefore evident the advantage represented by the fact that the tracheal aid **1** of the invention can realize both an overglottis aid and an endotracheal tube by modifying the arrangement of the component parts without having to remove them from the patient **P.** The two types of medical aids, therefore, are summarized in a single aid overcoming their incompatibility.

A further advantage is that this transition is reversible and can be repeated several times without restrictions in time and number of repetitions.

The operation more complicated during the above transition is to identify the trachea **T** with the fiberscope. Typically, during the intubation of a patient **P**, he is sedated and curarized. This treatment leads to a muscle paralysis and to the respiratory arrest. However, in the case of the aid 1 of the invention, this operation is performed during the assisted ventilation for which the patient **P** does not go in the apnea state during the search of the trachea **T** with the fiberscope, so allowing to increase the operation time while maintaining the safety of the patient **P** and while limiting the secretions and the regurgitation.

Moreover, in the prior art the use of the fiberscope after the use of curare or similar agents is contraindicated because the difficult of identifying the trachea **T** and the need to act quickly since the patient **P** in apnea state. In the case of the invention, on the contrary, there is not the urgency to proceed quickly because the operation is made during the assisted ventilation. The latter furthermore keeps open the air column between the respirator and the lungs making easy the identification of the trachea **T.**

Opportunely, the junction element **25** has softness and elasticity characteristics so as to avoid the irritation of the mucosa in the conjunction between the esophagus **E** and the trachea **T.**

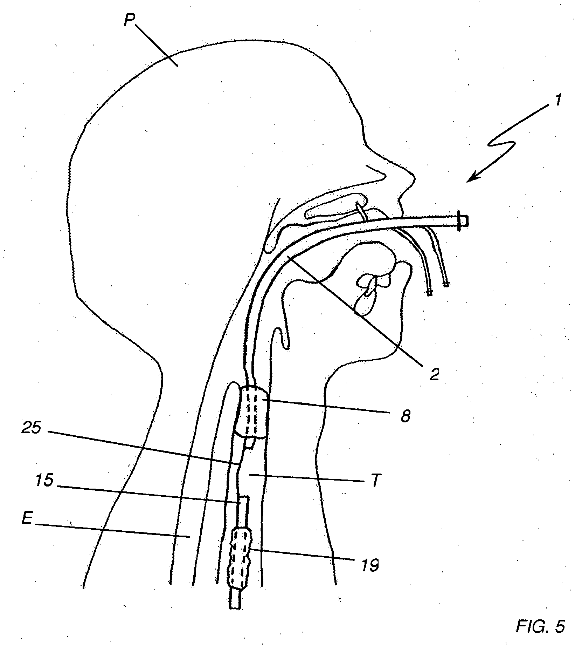

It has previously said that the insertion of the tracheal aid **1** of the invention if made without airways inspection have the consequence that the second tube **15,** with high probability, will be inserted into the esophagus **E.** However, there is a possibility, as represented in Fig. 4, of a fortuitous insertion of the second tube **15** into the trachea **T.** In this case also it is sufficient to increase the insertion of the first tube **2** so allowing the use of the tracheal aid 1 as an endotracheal tube, as shown in Fig. 5.

What emerges from what it has been said is that the tracheal aid **1** of the invention can be used always in the absence of inspection of the airways without error risk in the activation of the artificial respiration for the patient **P.** Moreover, this operation can be carried out by a non-expert user and even in critical situations for the patient **P.**

It follows that it is possible to avoid the use of the laryngoscope. Since this is the case for which it is necessary the use of curare, this allows to proceed simply sedating the patient **P.**

In the case of a severe trauma, the tracheal aid **1** of the invention allow to proceed starting on using it as an overglottis aid so as to immediately ventilate the patient **P** and then it is possible to proceed with intubation without to proceed quickly because the patient **P** is in a safe state. In particular, assisting a patient **P** it is possible to start applying the aid **1** as an overglottis aid and to proceed with the transition to an endotracheal tube only when the patient **P** is in a secure place.

With regard to the cuffs **8** and **19,** because their use typically the first cuff **8** has a maximum volume greater than the second cuff **19.** Moreover, the first cuff **8** is typically, but not necessarily, made by materials having an elastic coefficient higher than the material used for the second cuff **19.**

In the light of the foregoing, it is understood that the tracheal aid of the invention overcomes the drawbacks of the prior art being easy to apply on any patient in whatever state it is.

In particular, it can be successfully used by non-expert users and sum in a single aid, making them compatible, an overglottis aid and an endotracheal tube avoiding replacement/extraction procedures to switch from one to the other.

The aid of the invention also allows to protect the lungs from regurgitation or similars becoming from the esophagus during its use as an overglottis aid and also allows to avoid the apnea state for the patient in the transition from the overglottis configuration to the endotracheal configuration and vice versa.

The tracheal aid of the invention is susceptible of numerous modifications and variations, all falling within the inventive concept expressed in the appended claims. All the details may furthermore be replaced with other technically equivalent elements, and the materials may be different according to requirements, without departing from the scope of the invention.

Although the tracheal aid of the invention have been described with particular reference to the attached figures, reference numbers used in the description and in the claims are used to improve the intelligence of the invention and do not constitute any limitation to the scope of protection claimed.

## Claims

1. A tracheal aid usable for the assisted respiration of a patient comprising:
- at least one first tracheal tube **(2)** having a proximal end **(3)** disposable protruding from the mouth **(B)** of the patient **(P)** and connectable to a device for the assisted respiration, and a distal end **(5)** usable to be inserted on an orifice (**O**);
- at least one first inflatable cuff **(8)** externally coupled to said first tube **(2);**
- at least one second tracheal tube **(15)** having the distal end **(16)** usable to be inserted on an orifice (**O**);
- at least one second inflatable cuff **(19)** externally coupled to said second tube **(15);**
- at least one junction element **(25)** stably coupled to said first tube **(2)** and to said second tube **(15),**
**characterized in that** said junction element **(25)** is stably coupled with a distal end portion **(5)** of said first tube **(2)** and with a proximal end portion **(26)** of said second tube **(15)** so said first tube **(2),** said second tube **(15)** and said junction element **(25)** are substantially aligned, said junction element **(25)** being elastically flexible to enable said distal end **(5)** of said first tube **(2)** to be inserted on the trachea **(T)** of the patient **(P)** when said distal end **(16)** of said second tube **(15)** in inserted on the esophagus **(E)** of the patient **(P).**

2. Tracheal aid as claimed in claim 1, comprising at least one semirigid element placed internally to said first tube **(2)** and to said second tube **(15),** and removable from said position, to keep aligned said first tube **(2)** and second tube **(15).**

3. Tracheal aid as claimed in claim 2, **wherein** said semirigid element presents a system for indicating the penetration depth of said first tube **(2)** and of said second tube **(15).**

4. Tracheal aid as claimed in claim 3, **wherein** said indication system includes a graduated scale.

5. Tracheal aid as claimed in any of the preceding claims, comprising at least one first pipe **(9)** and at least one second pipe **(20)** distinct each other for the inflation of fluid inside each of said cuffs **(8, 19).**

6. Tracheal aid as claimed in any of the preceding claims, **wherein** said first cuff **(8)** is coupled to said first tube **(2)** near its distal end **(5).**

7. Tracheal aid as claimed in any of the preceding claims, **wherein** said first cuff **(8)** has a maximum inflation volume exceeding said second cuff **(19).**

8. Tracheal aid as claimed in any of the preceding claims, **wherein** said first cuff **(8)** has an elastic coefficient greater than said second cuff **(19).**

9. Tracheal aid as claimed in any of the preceding claims, comprising an extra-pipe that extends from said proximal end **(3)** of said first tube **(2)** to said distal end **(16)** of said second tube **(15)** for the transit of fluids between said two ends **(3, 16).**

## Patentansprüche

1. Tracheales Hilfsmittel zur Verwendung für die assistierte Beatmung eines Patienten, umfassend:
- mindestens einen ersten Trachealtubus **(2)** mit einem proximalen Ende **(3),** welches hervorstehend aus dem Mund **(B)** des Patienten **(P)** anordnenbar und mit einer Vorrichtung für die assistierte Beatmung verbindbar ist, und mit einem distalen Ende **(5),** welches für das Einsetzen in eine Öffnung **(O)** verwendbar ist;
- mindestens eine erste aufblasbare Manschette **(8),** die außenliegend mit dem ersten Tubus **(2)** verbunden ist;
- mindestens einen zweiten Trachealtubus **(15),** wobei das distale Ende **(16)** für das Einsetzen in eine Öffnung **(O)** verwendbar ist;
- mindestens eine zweite aufblasbare Manschette **(19),** die außenliegend mit dem zweiten Tubus **(15)** verbunden ist;
- mindestens ein Verbindungselement **(25),** das stabil mit dem ersten Tubus **(2)** und mit dem zweiten Tubus **(15)** verbunden ist,
**dadurch gekennzeichnet, dass** das Verbindungselement **(25)** stabil mit einem distalen Endabschnitt **(5)** des ersten Tubus **(2)** und mit einem proximalen Endabschnitt **(26)** des zweiten Tubus **(15)** verbunden ist, sodass der erste Tubus **(2),** der zweite Tubus **(15)** und das Verbindungselement **(25)** im Wesentlichen ausgerichtet sind, wobei das Verbindungselement **(25)** elastisch nachgiebig ist, um es zu ermöglichen, dass das distale Ende **(5)** des ersten Tubus **(2)** in die Luftröhre **(T)** des Patienten **(P)** eingeführt wird, wenn das distale Ende (16) des zweiten Tubus **(15)** in die Speiseröhre **(E)** des Patienten **(P)** eingeführt ist.

2. Tracheales Hilfsmittel nach Anspruch 1, umfassend mindestens ein halbsteifes Element, das innerhalb des ersten Tubus **(2)** und des zweiten Tubus **(15)** angeordnet und aus dieser Position entfernbar ist, um den ersten Tubus **(2)** und den zweiten Tubus **(15)** ausgerichtet zu halten.

3. Tracheales Hilfsmittel nach Anspruch 2, **wobei** das halbsteife Element ein System zum Anzeigen der Eindringtiefe des ersten Tubus **(2)** und des zweiten Tubus **(15)** darstellt.

4. Tracheales Hilfsmittel nach Anspruch 3, **wobei** das Anzeigesystem eine graduierte Skala umfasst.

5. Tracheales Hilfsmittel nach einem der vorhergehenden Ansprüche, umfassend mindestens ein erstes Rohr **(9)** und mindestens ein zweites Rohr **(20),** die sich voneinander unterscheiden, für die Befüllung von jeder der Manschetten **(8, 19)** mit Fluid.

6. Tracheales Hilfsmittel nach einem der vorhergehenden Ansprüche, **wobei** die erste Manschette **(8)** mit dem ersten Tubus (2) nahe an seinem distalen Ende **(5)** verbunden ist.

7. Tracheales Hilfsmittel nach einem der vorhergehenden Ansprüche, **wobei** die erste Manschette **(8)** ein maximales Aufblasvolumen aufweist, welches über das der zweiten Manschette **(19)** hinausgeht.

8. Tracheales Hilfsmittel nach einem der vorhergehenden Ansprüche, **wobei** die erste Manschette **(8)** einen Elastizitätskoeffizienten aufweist, der größer als derjenige der zweiten Manschette **(19)** ist.

9. Tracheales Hilfsmittel nach einem der vorhergehenden Ansprüche, umfassend ein zusätzliches Rohr, das sich von dem proximalen Ende **(3)** des ersten Tubus **(2)** zu dem distalen Ende **(16)** des zweiten Tubus **(15)** für den Durchgang von Fluiden zwischen den beiden Enden **(3, 16)** erstreckt.

## Revendications

1. Prothèse trachéale utilisable pour la respiration assistée d'un patient comprenant :
- au moins un premier tube trachéal (2) possédant une extrémité proximale (3) jetable dépassant de la bouche (B) du patient (P) et connectable à un dispositif pour la respiration assistée, et une extrémité distale (5) utilisable pour être insérée dans un orifice (O) ;
- au moins un premier manchon gonflable (8) couplé extérieurement audit premier tube (2) ;
- au moins un second tube trachéal (15) ayant l'extrémité distale (16) utilisable pour être insérée dans un orifice (O) ;
- au moins un second manchon gonflable (19) couplé extérieurement audit second tube (15) ;
- au moins un élément de jonction (25) couplé de manière stable audit premier tube (2) et audit second tube (15),
**caractérisée en ce que** ledit élément de jonction (25) est couplé de manière stable à une partie d'extrémité distale (5) dudit premier tube (2) et à une partie d'extrémité proximale (26) dudit second tube (15), afin que ledit premier tube (2), ledit second tube (15) et ledit élément de jonction (25) soient sensiblement alignés, ledit élément de jonction (25) étant flexible élastiquement pour permettre à ladite extrémité distale (5) dudit premier tube (2) d'être insérée dans la trachée (T) du patient (P) lorsque ladite extrémité distale (16) dudit second tube (15) est insérée dans l'oesophage (E) du patient (P).

2. Prothèse trachéale selon la revendication 1, comprenant au moins un élément semi-rigide placé intérieurement audit premier tube (2) et audit second tube (15), et pouvant être retiré de ladite position, pour maintenir alignés lesdits premier tube (2) et second tube (15).

3. Prothèse trachéale selon la revendication 2, dans laquelle ledit élément semi-rigide présente un système pour indiquer la profondeur de pénétration dudit premier tube (2) et dudit second tube (15).

4. Prothèse trachéale selon la revendication 3, dans laquelle ledit système d'indication comprend une échelle graduée.

5. Prothèse trachéale selon l'une quelconque des revendications précédentes, comprenant au moins un premier tuyau (9) et au moins un second tuyau (20) distincts l'un de l'autre permettant le gonflage de fluide à l'intérieur de chacun desdits manchons (8, 19).

6. Prothèse trachéale selon l'une quelconque des revendications précédentes, dans laquelle ledit premier manchon (8) est couplé audit premier tube (2) près de son extrémité distale (5).

7. Prothèse trachéale selon l'une quelconque des revendications précédentes, dans laquelle ledit premier manchon (8) possède un volume de gonflage maximum dépassant ledit second manchon (19).

8. Prothèse trachéale selon l'une quelconque des revendications précédentes, dans laquelle ledit premier manchon (8) possède un coefficient élastique supérieur audit second manchon (19).

9. Prothèse trachéale selon l'une quelconque des revendications précédentes, comprenant un tuyau supplémentaire qui s'étend de ladite extrémité proximale (3) dudit premier tube (2) à ladite extrémité distale (16) dudit second tube (15) pour le transit de fluides entre lesdites deux extrémités (3, 16).
